(19) **Europäisches Patentamt / European Patent Office / Office européen des brevets**

(11) **EP 2 008 604 B1**

(12) **EUROPEAN PATENT SPECIFICATION**

(45) Date of publication and mention
of the grant of the patent:
**26.12.2012 Bulletin 2012/52**

(51) Int Cl.:
***A61B 18/18*** *(2006.01)*

(21) Application number: **08011705.4**

(22) Date of filing: **27.06.2008**

(54) **Broadband microwave applicator**

Breitband-Mikrowellenapplikator

Applicateur à micro-ondes à large bande

(84) Designated Contracting States:
**DE ES FR GB IE IT**

(30) Priority: **28.06.2007 US 823639**

(43) Date of publication of application:
**31.12.2008 Bulletin 2009/01**

(73) Proprietor: **Vivant Medical, Inc.**
**Boulder CO 80301-3299 (US)**

(72) Inventor: **Brannan, Joseph**
**Boulder, CO 80302 (US)**

(74) Representative: **HOFFMANN EITLE**
**Patent- und Rechtsanwälte**
**Arabellastrasse 4**
**81925 München (DE)**

(56) References cited:
**WO-A-02/061880    WO-A-2004/112628**
**WO-A-2007/024878    WO-A-2007/076924**
**US-A- 5 275 597    US-A- 5 358 515**
**US-A- 5 904 709    US-A1- 2001 008 966**
**US-B1- 6 306 132**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## BACKGROUND

1. Technical Field

The present disclosure relates generally to microwave applicators used in tissue ablation procedures. More particularly, the present disclosure is directed to a microwave applicator having broadband matching performance over a band spanning a wide spectrum of operation frequencies.

2. Background of Related Art

Treatment of certain diseases requires destruction of malignant tissue growths (e.g., tumors). It is known that tumor cells denature at elevated temperatures that are slightly lower than temperatures injurious to surrounding healthy cells. Therefore, known treatment methods, such as hyperthermia therapy, heat tumor cells to temperatures above 41° C, while maintaining adjacent healthy cells at lower temperatures to avoid irreversible cell damage. Such methods involve applying electromagnetic radiation to heat tissue and include ablation and coagulation of tissue. In particular, microwave energy is used to coagulate and/or ablate tissue to denature or kill the cancerous cells.

Microwave energy is applied via microwave ablation antenna probes which penetrate tissue to reach tumors. There are several types of microwave probes, such as monopole and dipole. In monopole and dipole probes, microwave energy radiates perpendicularly from the axis of the conductor. Monopole probe (e.g., antenna) includes a single, elongated microwave conductor. Dipole probes have a coaxial construction including an inner conductor and an outer conductor separated by a dielectric portion. More specifically, dipole microwave antennas have a long, thin inner conductor which extends along a longitudinal axis of the probe and is surrounded by an outer conductor. In certain variations, a portion or portions of the outer conductor may be selectively removed to provide for more effective outward radiation of energy. This type of microwave probe construction is typically referred to as a "leaky waveguide" or "leaky coaxial" antenna.

Conventional microwave probes have a narrow operational bandwidth, a wavelength range at which optimal operational efficiency is achieved, and hence, are incapable of maintaining a predetermined impedance match between the microwave delivery system (e.g., generator, cable, etc.) and the tissue surrounding the microwave probe. More specifically, as microwave energy is applied to tissue, the dielectric constant of the tissue immediately surrounding the microwave probe decreases as the tissue is cooked. The drop causes the wavelength of the microwave energy being applied to tissue to increase beyond the bandwidth of the probe. As a result, there is a mismatch between the bandwidth of conventional microwave probe and the microwave energy being applied. Thus, narrow band microwave probes may detune hindering effective energy delivery and dispersion.

## SUMMARY

The invention is defined in the independent claims below. The dependent claims are directed to optional features and preferred embodiments.

The present disclosure provides for a microwave ablation probe configured to maintain an impedance match to impedance of a microwave energy delivery system (e.g., microwave generator, cable, etc.) despite tissue state changes encountered during the course of the microwave ablation as the dielectric constant of the tissue changes. The microwave ablation probe is passively broadband in nature (e.g., a band spanning 40% of the frequency of the microwave energy) due to the structure of the probe. In embodiments, the probe includes either a balanced or an unbalanced coaxial fed dipole antenna having one or more capacitive metallic disks and/or dielectric materials at a radiation portion of the probe. For examples of such a probe see US-A-5 275 597, US-A-5 904 709 and WO-A-2004/112628. In other embodiments, the probe includes the so-called "folded-dipole" antenna disposed in the radiation portion of the probe which is also loaded with dielectric materials.

A microwave ablation probe for providing microwave energy to tissue is disclosed. The probe includes a feedline having an inner conductor, an insulating spacer and an outer conductor. The probe also includes a radiation portion having an extruded portion of the inner conductor that is centrally disposed therein. The radiation portion also includes one or more conductive disks disposed on the extruded portion of the inner conductor that defines one or more spaces and a dielectric material disposed within the spaces.

In another embodiment, the probe includes a radiation portion including a folded-dipole antenna constructed from an extruded portion of the inner conductor and at least one outer arm coupled to the outer conductor. The radiation portion further includes a dielectric material disposed around the folded-dipole antenna.

A microwave ablation probe for providing microwave energy to tissue is disclosed, which includes a radiation portion having an extruded portion of the inner conductor that is centrally disposed therein. The radiation portion also includes one or more disks disposed on the extruded portion of the inner conductor that define one or more corresponding spaces. The radiation portion also includes one or more supply tubes configured to supply a liquid cooling dielectric material into the spaces.

## BRIEF DESCRIPTION OF THE DRAWINGS

The above and other aspects, features, and advantages of the present disclosure will become more apparent in light of the following detailed description when taken in conjunction with the accompanying drawings in which:

Fig. 1 is a schematic diagram of a microwave ablation system which does not include the liquid cooling dielectric material of the invention claimed below;

Fig. 2 is a perspective cross-sectional view of the microwave ablation probe;

Fig. 3 is a perspective view (part disassembled) of the microwave ablation probe of Fig. 2.

Fig. 4 is a side cross-sectional view of the microwave ablation probe of Fig. 2;

Fig. 5 is a perspective cross-sectional view of a first embodiment of the microwave ablation probe having an supply duct according to the invention claimed below; and

Fig. 6 is a perspective cross-sectional view of a second embodiment of the microwave ablation probe having an supply duct according to the invention claimed below.

## DETAILED DESCRIPTION

In the following description, well-known functions or constructions are not described in detail to avoid obscuring the present disclosure in unnecessary detail.

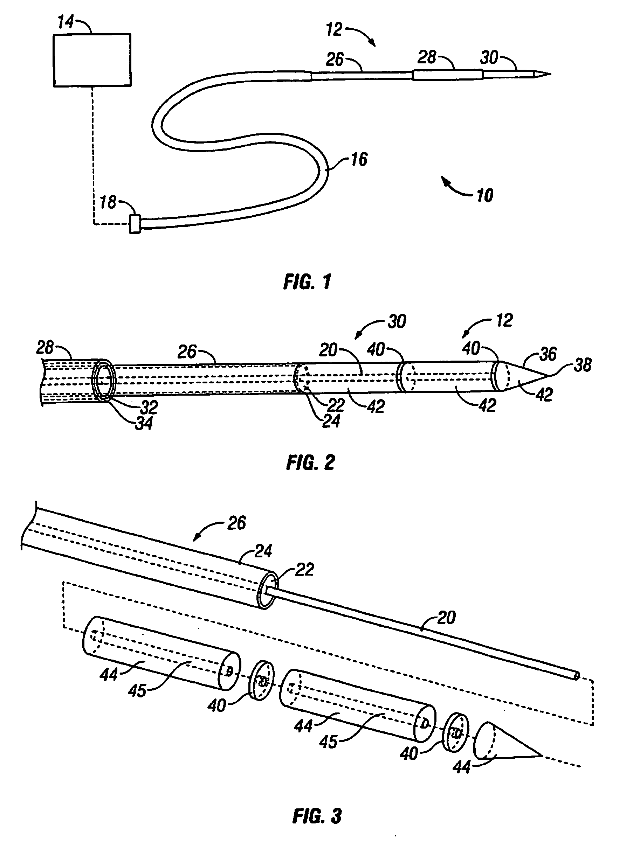

Fig.1 shows a microwave ablation system 10 which includes a microwave ablation probe 12 coupled to a microwave generator 14 via a flexible coaxial cable 16, which is, in turn, coupled to a connector 18 of the generator 14. The generator 14 is configured to provide microwave energy at an operational frequency from about 500 MHz to about 2500 MHz.

WO-A-2007/024878 discloses use of air, gas or fluid in a coaxial cable carrying microwave power to serve as a dielectric and to dissipate heat.

During microwave ablation, the probe 12 is inserted into tissue and microwave energy is supplied thereto. As tissue surrounding the probe 12 is ablated, the tissue undergoes desiccation and denaturization which results in a drop of the effective dielectric constant of the tissue (i.e., increase in impedance). The drop in the effective dielectric constant, in turn, lengthens the wavelength of the microwave energy. Since the probe length is held constant during ablation, the increase in the wavelength results in the increase of the optimal operational frequency of the probe. Thus, at the outset the probe 12 is at an initial match point - a predetermined operational frequency that increases to a higher frequency as the ablation continues. The higher frequency is determined according to the formula (1), wherein ($\varepsilon_r$) is the dielectric constant and f is the frequency:

$$(1) \quad \sqrt{(\varepsilon_{r\,uncooked} / \varepsilon_{r\,cooked})}\; f_{oper} = f_{high}$$

With respect to liver tissue, in a normal uncooked state, the liver tissue has a dielectric constant of 50 with the operational frequency being 915 MHz. In a cooked state, the liver tissue has a dielectric constant of 25. Substituting these values into the formula (1) provides the lower frequency, which in this case is 1300 MHz. The probe 12 according to the present disclosure has an operational bandwidth configured to encompass the initial match point as well as the higher frequency. In particular, the bandwidth of the probe 12 is approximately 40% of the operational frequency. In embodiments, the probe 12 is loaded with one or more of the following: one or more disks, liquid and/or solid dielectric materials. These materials provide a static envelope around the antenna and act as a buffer between the antenna and the tissue. Use of a liquid dielectric material also allows for active cooling of the antenna during ablation in addition to providing a dielectric buffer.

As shown in Figs. 2-4, the probe 12 includes a feedline 26, a choke 28 and a radiating portion 30. The feedline 26 extends between the distal end of the probe 12 where the feedline 26 is coupled to the cable 16, to the radiating portion 30. The feedline 26 is constructed from a coaxial cable having an inner conductor 20 (e.g., wire) surrounded by an insulating spacer 22 which is then surrounded by an outer conductor 24 (e.g., cylindrical conducting sheath). In one embodiment, the feedline 26 may have a diameter of 2.16 mm (0.085 inches) and the insulating spacer 22 may have a dielectric constant of 1.7.

The feedline 26 may be flexible or semi-rigid and may be of variable length from a proximal end of the radiating portion 30 to a distal end of the cable 16 ranging from about 25 to about 254 mm (from about 1 to about 10 inches). The inner conductor 20 and the outer conductor 24 may be constructed from a variety of metals and alloys, such as copper, gold, stainless steel, and the like. Metals may be selected based on a variety of factors, such as conductivity and tensile strength. Thus, although stainless steel has lower conductivity than copper and/or gold, stainless steel provides the necessary strength required to puncture tissue and/or skin. In such cases, the inner and outer conductors 20 and 24 may be plated with conductive material (e.g., copper, gold, etc.) to improve conductivity and/or decrease energy loss.

The choke 28 of the probe 12 is disposed around the feedline 26 and includes an inner dielectric layer 32 and an outer conductive layer 34. The choke 28 confines the microwave energy from the generator 14 to the radiating portion 30 of the probe 12 thereby limiting the microwave energy deposition zone length along the feedline 26. The choke 28 is implemented with a quarter wave short by using the outer conductive layer 34 around the outer conductor 24 of the feedline 26 separated by the dielectric layer 32. The choke 28 is shorted to the outer conductor 24 of the feedline 26 at the proximal end of the choke 28 by soldering or other means. In embodiments, the length of the choke 28 may be from a quarter to a full wavelength. The choke 28 acts as a high impedance to microwave energy conducted down the outside of the feedline 26 thereby limiting energy deposition to the end of the probe. In the illustrated probe, the dielectric layer 32 is formed from a fluoropolymer such as tetrafluorethylene, perfluorpropylene, and the like and has a thickness of 0.13 mm (0.005 inches). The outer conductive layer 34 may be formed from a so-called "perfect conductor" material such as a highly conductive metal (e.g., copper).

The probe 12 further includes a tapered end 36 which terminates in a tip 38 at the distal end of the radiating portion 30. The tapered end 36 allows for insertion of the probe 12 into tissue with minimal resistance. In cases where the radiating portion 12 is inserted into a pre-existing opening, the tip 38 may be rounded or flat. The tapered end 36 may be formed from any type of hard material such as metal and/or plastic.

The probe 12 shown in Figs. 2-4 includes one or more conductive disks 40 loaded therein. The feedline 26 extends past the distal end of the choke 28, with the insulating spacer 22 and the outer conductor 24 terminating at the start of the radiating portion 30. The inner conductor 20 is extruded from the feedline 26 and extends into the radiating portion 30 where the inner conductor 20 is centrally disposed. The extruded portion of the inner conductor 20 includes one or more of the conductive disks 40 which are also centrally disposed thereon (i.e., the center of the disks 40 is on the longitudinal axis). The disks 40 are perpendicular to a longitudinal axis defined by the inner conductor 20. In one embodiment, the disks 40 have a thickness from about 0.25 to about 0.50 mm (from about 0.01 inches to about 0.02 inches) and have a diameter from about 1 mm (0.04 inches) to about the thickness of the feedline 26, which in one embodiment is 2.16 mm (0.085 inches). The disks 40 may be of varying size, diameter and thickness, or all of the disks 40 may be of the same size. The disks 40 are spaced on the inner conductor 20 such that the desired bandwidth is obtained.

The disks 40 divide the radiating portion 30 into a corresponding number of spaces 42: the spaces 42 between the feedline 26 and the first disk 40, between the first and second disks 40, and within the tapered end 36. The spaces 42 are loaded with a solid dielectric material 44 which is shaped to fill the corresponding spaces 40 to further improve the impedance match between the probe 12 and the generator 14. More specifically, to fill the spaces 42 between the disks 40, the material 44 may be cylinder-shaped having a central cavity 45 defined therein as illustrated in Fig. 3. The cylinder has an outer diameter being substantially equal to the thickness of the feedline 26 and the inner diameter being substantially equal to the diameter of the inner conductor 20. To fill the space 42 at the tapered end 36, the material 44 may be cone-shaped. In one embodiment, the material 44 has a dielectric constant of from about 2.5 and 30 and may be made from a ceramic material, such as alumina ceramic or a plastic material, such as a polyamide plastic (e.g., VESPEL® available from DuPont of Wilmington, Delaware).

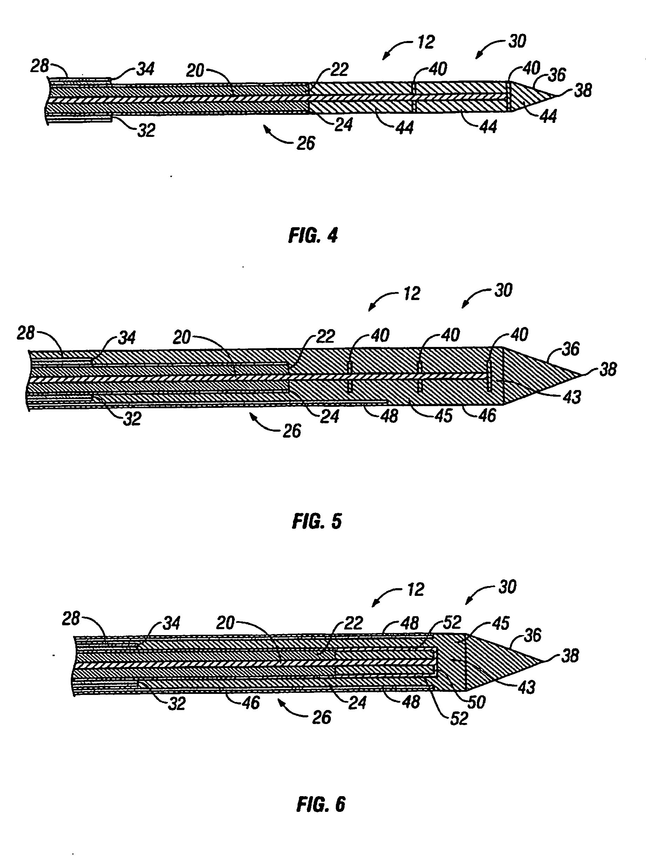

Fig. 5 illustrates a first embodiment of the claimed microwave ablation probe 12. The probe 12 includes the radiating portion 30 coupled to the feedline 26 which is covered by the choke 28. The feedline 26 includes the inner conductor 20 surrounded by the insulating spacer 22 which is then surrounded by the outer conductor 24. The inner conductor 20 of the feedline 26 includes one or more disks 40 perpendicularly disposed thereon. The feedline 26, at least a portion of the choke 28, and the radiating portion 30 are enclosed within a cavity 43 formed by a moisture-impervious housing 46. The probe 12 also includes one or more supply tubes 48 that supply a dielectric fluid 45, such as water and the like, into the cavity 43. The dielectric fluid 45 provides for an impedance match as well as cools the probe 12. The dielectric fluid 45 may be stored in a supply tank (not explicitly shown) and may be supplied by a pump (e.g., peristaltic pump) into the cavity 43. The supply tube 48 is constructed from a flexible material such as polyamide polymer.

Fig. 6 shows a second embodiment of the probe 12 which includes the choke 28 enclosing the feedline disposed within the radiating portion 30. The radiation portion 30 includes an unbalanced folded-dipole antenna 50 which includes the extruded inner conductor 20 as a central arm and one or more outer arms 52 which extend from and are coupled to the distal end of inner conductor 20. The outer arms 52 are also coupled to the outer conductor 24 of the feedline 26. The outer arms 52 are coupled to the inner conductor 20 and the outer conductor 24 by soldering and/or other methods which allow for conductive coupling of metals. The length of the folded-dipole antenna 50 is between a quarter and a full wavelength of the operating microwave energy, effectively providing for an optimum impedance match.

The radiation portion 30, the feedline 26 and the choke 28 are disposed within the cavity 43 defined by the housing 46. The cavity 43 also includes one or more supply tubes 48 which supply the fluid 45 thereto. In embodiments shown in Figs. 5 and 6, the dielectric fluid 45 may be circulated through the cavity 43 by continually supplying the fluid 45 through the supply tube 48 and withdrawing the fluid using a return tube (not explicitly shown).

The probe 12 according to the present disclosure has a broadband range encompassing the frequency variation encountered during ablation due to tissue state changes. The probe 12 is configured to maintain an impedance match to the generator 14 and the cable 16 which provides for improved microwave deposition and penetration depth that are maintained throughout the course of an ablation despite tissue changes.

The described embodiments of the present disclosure are intended to be illustrative rather than restrictive, and are not intended to represent every embodiment of the present disclosure. Various modifications and variations can be made without departing from the scope of the claims which follow.

## Claims

1. A microwave ablation probe (12) for providing microwave energy to tissue, the probe comprising:

   a feedline (26) including an inner conductor (20), an insulating spacer (22) and an outer conductor (24);
   a radiation portion (30) including at least a portion of the inner conductor that is centrally disposed therein, the radiation portion including at least one conductive disk (40)disposed on the portion of the inner conductor that defines at least one space; **characterised by**
   a liquid cooling dielectric material disposed within the at last one space; and
   at least one supply tube configured to supply the liquid cooling dielectric material into the at least one space.

2. A microwave ablation probe for providing microwave energy to tissue, the probe comprising:

   a feedline including an inner conductor, and insulating spacer and an outer conductor;
   a radiation portion including a folded-dipole antenna constructed from at least a portion of the inner conductor and at last one outer arm coupled to the outer conductor,
   **characterised by**
   the radiation portion further including a liquid cooling dielectric material disposed around the folded-dipole antenna; and
   at least one supply tube configured to supply the liquid cooling dielectric material into the radiation portion.

3. A microwave ablation probe according to claim 1 or 2, further including a choke (28) disposed around at least a portion of the feedline and configured to confine the microwave energy to the radiating portion, the choke including an inner dielectric layer (32) and an outer conductive layer (34).

4. A microwave ablation probe according to any preceding claim, further including a tapered end (36) having a tip (38) disposed at a distal end of the radiating portion.

5. A microwave ablation probe according to any one of the preceding claims, wherein the liquid cooling dielectric material is water.

## Patentansprüche

1. Mikrowellenablationssonde (12) zum Bereitstellen von Mikrowellenenergie an Gewebe, die Sonde mit:

   einer Zuführleitung (26), die einen inneren Leiter (20), ein Isolationsabstandsstück (22) und einen äußeren Leiter (24) aufweist;
   einem Strahlabschnitt (30), der zumindest einen Abschnitt des inneren Leiters aufweist, der mittig darin angeordnet ist, wobei der Strahlabschnitt mindestens eine leitfähige Scheibe (40) aufweist, die auf dem Abschnitt des inneren Leiters angeordnet ist, der mindestens einen Raum definiert;
   **gekennzeichnet durch**
   ein flüssiges dielektrisches Kühlmaterial, das in dem mindestens einen Raum angeordnet ist; und
   mindestens eine Zuführröhre, die eingerichtet ist, das flüssige dielektrische Kühlmaterial in den mindestens einen Raum zu führen.

2. Mikrowellenablationssonde zum Bereitstellen von Mikrowellenenergie an Gewebe, die Sonde mit:

   einer Zuführleitung, die einen inneren Leiter, ein Isolationsabstandsstück und einen äußeren Leiter aufweist;
   einem Strahlabschnitt, der eine Faltdipolantenne aufweist, die aus mindestens einem Abschnitt des inneren Leiters und mindestens einem mit dem äußeren Leiter gekoppelten äußeren Arm zusammengesetzt ist,
   **dadurch gekennzeichnet,**
   **dass** der Strahlabschnitt des Weiteren ein flüssiges dielektrisches Kühlmaterial aufweist, das um die Faltdipolantenne angeordnet ist; und
   mindestens eine Zuführröhre, die eingerichtet ist, das flüssige dielektrische Kühlmaterial in den Strahlabschnitt zu führen.

3. Mikrowellenablationssonde nach Anspruch 1 oder 2, des Weiteren mit einer Drossel (28), die um mindestens einen Abschnitt der Zuführleitung angeordnet und eingerichtet ist, die Mikrowellenenergie auf den Strahlabschnitt zu beschränken, wobei die Drossel eine innere dielektrische Schicht (32) und eine äußere leitfähige Schicht (34) aufweist.

4. Mikrowellenablationssonde nach einem der vorstehenden Ansprüche, des Weiteren mit einem sich verjüngenden Ende (36), das eine an einem distalen Ende des Strahlabschnitts angeordnete Spitze (38) aufweist.

5. Mikrowellenablationssonde nach einem der vorstehenden Ansprüche, bei der das flüssige dielektrische Kühlmaterial Wasser ist.

## Revendications

1. Sonde d'ablation à micro-ondes (12) pour fournir de l'énergie de micro-ondes à du tissu, la sonde comprenant :

   une ligne d'amenée (26) incluant un conducteur intérieur (20), une pièce d'écartement isolante (22) et un conducteur extérieur (24) ;
   une portion de rayonnement (30) incluant au moins une portion du conducteur intérieur qui est disposée au centre dans celle-ci, la portion de rayonnement incluant au moins un disque conducteur (40) disposé sur la portion du conducteur intérieur qui définit au moins un espace ;
   **caractérisée par** un matériau diélectrique de refroidissement liquide disposé dans le au moins un espace ; et
   au moins un tube d'amenée configuré pour amener le matériau diélectrique de refroidissement liquide dans le au moins un espace.

2. Sonde d'ablation à micro-ondes pour fournir de l'énergie de micro-ondes à du tissu, la sonde comprenant :

   une ligne d'amenée incluant un conducteur intérieur, et une pièce d'écartement isolante et un conducteur extérieur ;
   une portion de rayonnement incluant une antenne dipôle pliée construite à partir d'au moins une portion du conducteur intérieur et d'au moins un bras extérieur couplé au conducteur extérieur, **caractérisée en ce que** la portion de rayonnement comprend en outre un matériau diélectrique de refroidissement liquide disposé autour de l'antenne dipôle pliée ; et
   au moins un tube d'amenée configuré pour amener le matériau diélectrique de refroidissement liquide dans la portion de rayonnement.

3. Sonde d'ablation à micro-ondes selon la revendication 1 ou 2, incluant en outre une bobine d'arrêt (28) disposée autour d'au moins une portion de la ligne d'amenée et configurée pour confiner l'énergie micro-ondes à la portion de rayonnement, la bobine d'arrêt incluant une couche diélectrique intérieure (32) et une couche conductrice extérieure (34).

4. Sonde d'ablation à micro-ondes selon l'une quelconque des revendications précédentes, comprenant en outre une extrémité effilée (36) comportant une pointe (38) disposée à une extrémité distale de la portion de rayonnement.

5. Sonde d'ablation à micro-ondes selon l'une quelconque des revendications précédentes, dans laquelle le matériau diélectrique de refroidissement liquide est de l'eau.

**FIG. 1**

**FIG. 2**

**FIG. 3**

7

**FIG. 4**

**FIG. 5**

**FIG. 6**

**REFERENCES CITED IN THE DESCRIPTION**

**Patent documents cited in the description**

- US 5275597 A
- US 5904709 A
- WO 2004112628 A
- WO 2007024878 A